# EUROPÄISCHE PATENTANMELDUNG

(11) **EP 1 205 553 A1**
(43) Veröffentlichungstag der Anmeldung: **15.05.2002**
(21) Anmeldenummer: 01117264.0
(22) Anmeldetag: 17.07.2001
(51) Int. Cl.: C12N 15/54, C12N 9/12, C12N 1/21, C07K 14/34, C12Q 1/68, C12P 13/00

(54) **Für das sigD-Gen kodierende Nukleotidsequenzen**

(30) Priorität: 02.09.2000 DE 10043331
(71) Anmelder: Degussa AG, 40474 Düsseldorf (DE)
(72) Erfinder: Bathe, Brigitte, Dr., 33154 Salzkotten (DE); Kreutzer, Caroline, 49326 Melle (DE); Martens, Monika, 33719 Bielefeld (DE); Farwick, Mike, Dr., 33515 Bielefeld (DE); Hermann, Thomas, Dr., 33739 Bielefeld (DE); Pfefferle, Walter, Dr., 33790 Halle (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
und ein Verfahren zur fermentativen Herstellung von L-Aminosäuren unter Verwendung von coryneformen Bakterien, in denen zumindest das sigD-Gen verstärkt vorliegt, und die Verwendung von Polynukleotiden, die die erfindungsgemäßen Sequenzen enthalten, als Hybridisierungssonden.

## Beschreibung

Gegenstand der Erfindung sind für das sigD-Gen kodierende Nukleotidsequenzen aus coryneformen Bakterien und ein Verfahren zur fermentativen Herstellung von Aminosäuren unter Verwendung von Bakterien, in denen mindestens das endogene sigD-Gen verstärkt wird.

### Stand der Technik

L-Aminosäuren finden in der Humanmedizin und in der pharmazeutischen Industrie, in der Lebensmittelindustrie und ganz besonders in der Tierernährung, Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht.

### Aufgabe der Erfindung

Die Erfinder haben sich zur Aufgabe gestellt, neue Maßnahmen zur verbesserten fermentativen Herstellung von Aminosäuren bereitzustellen.

### Beschreibung der Erfindung

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin gemeint. Besonders bevorzugt ist Lysin.

Gegenstand der Erfindung ist ein isoliertes Polynukleotid aus coryneformen Bakterien, enthaltend eine für das sigD-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
wobei das Polypeptid bevorzugt die Aktivität des Sigma-Faktors D aufweist.

Gegenstand der Erfindung ist ebenfalls das oben genannte Polynukleotid, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein replizierbares Polynukleotid, insbesondere DNA, enthaltend die Nukleotidsequenz wie in SEQ ID No. 1 dargestellt;
ein Polynukleotid, das für ein Polypeptid kodiert, das die Aminosäuresequenz, wie in SEQ ID No. 2 dargestellt, enthält;
ein Vektor, enthaltend das erfindungsgemäße Polynukleotid, insbesondere Pendelvektor oder Plasmidvektor, und
coryneforme Bakterien, die den Vektor enthalten oder in denen das sigD-Gen verstärkt ist.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank eines coryneformen Bakteriums, die das vollständige Gen oder Teile davon enthält, mit einer Sonde, die die Sequenz des erfindungsgemäßen Polynukleotids gemäß SEQ ID No.1 oder ein Fragment davon enthält und Isolierung der genannten Polynukleotidsequenz.

Polynukleotide, die die Sequenzen gemäß der Erfindung enthalten, sind als Hybridisierungs-Sonden für RNA, cDNA und DNA geeignet, um Nukleinsäuren beziehungsweise Polynukleotide oder Gene in voller Länge zu isolieren, die für den Sigma-Faktor D kodieren, oder um solche Nukleinsäuren beziehungsweise Polynukleotide oder Gene zu isolieren, die eine hohe Ähnlichkeit der Sequenz mit der des sigD-Gens aufweisen. Sie sind ebenso zum Einbau in sogenannte "arrays", "micro arrays" oder "DNA chips" geeignet, um die entsprechenden Polynukleotide zu detektieren und zu bestimmen.

Polynukleotide, die die Sequenzen gemäß der Erfindung enthalten, sind weiterhin als Primer geeignet, mit deren Hilfe mit der Polymerase-Kettenreaktion (PCR) DNA von Genen hergestellt werden kann, die für den Sigma-Faktor D kodieren.

Solche als Sonden oder Primer dienende Oligonukleotide enthalten mindestens 25, 26, 27, 28, 29 oder 30, bevorzugt mindestens 20, 21, 22, 23 oder 24 ganz besonders bevorzugt mindestens 15, 16, 17, 18 oder 19 aufeinanderfolgende Nukleotide. Geeignet sind ebenfalls Oligonukleotide mit einer Länge von mindestens 31, 32, 33, 34, 35, 36, 37, 38, 39 oder 40 oder mindestens 41, 42, 43, 44, 45, 46, 47, 48, 49 oder 50 Nukleotiden. Gegebenenfalls sind auch Oligonukleotide mit einer Länge von mindestens 100, 150, 200, 250 oder 300 Nukleotiden geeignet.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Die Polynukleotide gemäß Erfindung schließen ein Polynukleotid gemäß SEQ ID No. 1 oder ein daraus hergestelltes Fragment und auch solche ein, die zu wenigstens 70% bis 80%, bevorzugt zu wenigstens 81% bis 85%, besonders bevorzugt zu wenigstens 86% bis 90%, und ganz besonders bevorzugt zu wenigstens 91%, 93%, 95%, 97% oder 99% identisch sind mit dem Polynukleotid gemäß SEQ ID No. 1 oder eines daraus hergestellten Fragments.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität des Sigma-Faktors D und auch solche ein, die zu wenigstens 70% bis 80%, bevorzugt zu wenigstens 81% bis 85%, besonders bevorzugt zu wenigstens 91%, 93%, 95%, 97% oder 99% identisch mit dem Polypeptid gemäß SEQ ID No. 2 sind und die genannte Aktivität aufweisen.

Die Erfindung betrifft weiterhin ein Verfahren zur fermentativen Herstellung von Aminosäuren, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin, unter Verwendung von coryneformen Bakterien, die insbesondere bereits Aminosäuren produzieren und in denen die für das sigD-Gen kodierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum (C. glutamicum), sind besonders die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme.

Den Erfindern gelang es, das neue, für das Enzym Sigma-Faktor D kodierende sigD-Gen von C. glutamicum zu isolieren.

Zur Isolierung des sigD-Gens oder auch anderer Gene von C. glutamicum wird zunächst eine Genbank dieses Mikroorganismus in Escherichia coli (E. coli) angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990), oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde.

Börmann et al. (Molecular Microbiology 6(3), 317-326) (1992)) wiederum beschreiben eine Genbank von C. glutamicum ATCC13032 unter Verwendung des Cosmids pHC79 (Hohn und Collins, Gene 11, 291-298 (1980)).

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem von Marck (Nucleic Acids Research 16, 1829-1836 (1988)) oder dem GCG-Programm von Butler (Methods of Biochemical Analysis 39, 74-97 (1998)) untersucht werden.

Auf diese Weise wurde die neue für das Gen sigD kodierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des sigD-Genproduktes dargestellt.

Kodierende DNA-Sequenzen, die sich aus SEQ ID No. 1 durch die Degeneriertheit des genetischen Kodes ergeben, sind ebenfalls Bestandteil der Erfindung. In gleicher Weise sind DNA-Sequenzen, die mit SEQ ID No. 1 oder Teilen von SEQ ID No. 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d.h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID No. 2 ergeben, sind ebenfalls Bestandteil der Erfindung.

In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heisst, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflußt bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur von ca. 50 - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50 - 68°C eingestellt wird. Es ist gegebenenfalls möglich die Salzkonzentration bis auf 0,1x SSC zu senken. Durch schrittweise Erhöhung der Hybridisierungstemperatur in Schritten von ca. 1 - 2°C von 50 auf 68°C können Polynukleotidfragmente isoliert werden, die beispielsweise mindestens 70% oder mindestens 80% oder mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994).

Bei der Arbeit an der vorliegenden Erfindung konnte festgestellt werden, daß coryneforme Bakterien nach Überexpression des endogenen sigD-Gens in verbesserter Weise Aminosäuren produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Zur Verstärkung wurde das erfindungsgemäße sigD-Gen beispielhaft mit Hilfe von episomalen Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Zusätzlich kann es für die Produktion von L-Aminosäuren vorteilhaft sein, neben dem sigD-Gen eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine zu verstärken, insbesondere überzuexprimieren.

So kann beispielsweise für die Herstellung von L-Aminosäuren zusätzlich zur Verstärkung des sigD-Gens eines oder mehrere Gene, ausgewählt aus der Gruppe
- das für die Dihydrodipicolinat-Synthase kodierende Gen dapA (EP-B 0 197 335),
- das für die Glyceraldehyd-3-Phosphat Dehydrogenase kodierende Gen gap (Eikmanns (1992), Journal of Bacteriology. 174:6076-6086),
- das für die Triosephosphat Isomerase kodierende Gen tpi (Eikmanns (1992), Journal of Bacteriology 174:6076-6086),
- das für die 3-Phosphoglycerat Kinase kodierende Gen pgk (Eikmanns (1992), Journal of Bacteriology 174:6076-6086),
- das für die Glucose-6-Phosphat Dehydrogenase kodierende Gen zwf (JP-A-09224661),
- das für die Pyruvat Carboxylase kodierende Gen pyc (DE-A-198 31 609),
- das für die Malat-Chinon-Oxidoreduktase kodierende Gen mqo (Molenaar et al., European Journal of Biochemistry 254, 395-403 (1998)),
- das für eine feed-back resistente Aspartatkinase kodierende Gen lysC (Accession No.P26512),
- das für den Lysin-Export kodierende Gen lysE (DE-A-195 48 222),
- das für die Homoserin-Dehydrogenase kodierende Gen hom (EP-A 0131171),
- das für die Threonin-Dehydratase kodierende Gen ilvA (Möckel et al., Journal of Bacteriology (1992) 8065-8072)) oder das für eine "feed back resistente" Threonin-Dehydratase kodierende Allel ilvA(Fbr) (Möckel et al., (1994) Molecular Microbiology 13: 833-842),
- das für die Acetohydroxysäure-Synthase kodierenden Gen ilvBN (EP-B 0356739),
- das für die Dihydroxysäuredehydratase kodierende Gen ilvD (Sahm und Eggeling (1999) Applied and Environmental Microbiology 65: 1973-1979),
- das für das Zwa1-Protein kodierende Gen zwa1 (DE: 19959328.0, DSM 13115)
verstärkt, insbesondere überexprimiert werden.

Weiterhin kann es für die Produktion von L-Aminosäuren vorteilhaft sein, zusätzlich zur Verstärkung des sigD-Gens eines oder mehrere Gene, ausgewählt aus der Gruppe
- das für die Phosphoenolpyruvat-Carboxykinase kodierende Gen pck (DE 199 50 409.1; DSM 13047),
- das für die Glucose-6-Phosphat Isomerase kodierende Gen pgi (US 09/396,478; DSM 12969),
- das für die Pyruvat-Oxidase kodierende Gen poxB (DE: 1995 1975.7; DSM 13114),
- das für das Zwa2-Protein kodierende Gen zwa2 (DE: 19959327.2, DSM 13113)
abzuschwächen, insbesondere die Expression zu verringern.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Massnahmen kombiniert.

Weiterhin kann es für die Produktion von Aminosäuren vorteilhaft sein, neben der Überexpression des sigD-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B.

Antibiotika hinzugefügt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Ionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Das erfindungsgemäße Verfahren dient zur fermentativen Herstellung von Aminosäuren.

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular Cloning. A Laboratory Manual (1989) Cold Spring Harbour Laboratory Press, Cold Spring Harbor, NY, USA) durchgeführt. Methoden zur Transformation von Escherichia coli sind ebenfalls in diesem Handbuch beschrieben.

Die Zusammensetzung gängiger Nährmedien wie LB- oder TY-Medium kann ebenfalls dem Handbuch von Sambrook et al. entnommen werden.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Diagnostics GmbH, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert.

Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt.

Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 mg/l Ampicillin ausplattiert wurden. Nach Inkubation über Nacht bei 37°C wurden rekombinante Einzelklone selektioniert.

### Beispiel 2

### Isolierung und Sequenzierung des sigD-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Diagnostics GmbH, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany).

Die DNA des Sequenziervektors pZero-1, bezogen von der Firma Invitrogen (Groningen, Niederlande, Produktbeschreibung Zero Background Cloning Kit, Product No. K2500-01), wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A Laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l Zeocin ausplattiert.

Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurde mit dem Programm XNIP (Staden, 1986, Nucleic Acids Research, 14:217-231) angefertigt.

Die erhaltene Nukleotidsequenz ist in SEQ ID No. 1 dargestellt. Die Analyse der Nukleotidsequenz ergab ein offenes Leseraster von 567 Basenpaaren, welches als sigD-Gen bezeichnet wurde. Das sigD-Gen kodiert für ein Protein von 188 Aminosäuren.

## Patentansprüche

**1.** Isoliertes Polynukleotid aus coryneformen Bakterien, enthaltend eine für das sigD-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
wobei das Polypeptid bevorzugt die Aktivität des Sigma-Faktors D aufweist.

**2.** Polynukleotid gemäß Anspruch 1, wobei das Polynukleotid eine in coryneformen Bakterien replizierbare, bevorzugt rekombinante DNA ist.

**3.** Polynukleotid gemäß Anspruch 1, wobei das Polynukleotid eine RNA ist.

**4.** Polynukleotid gemäß Anspruch 2, enthaltend die Nukleinsäuresequenz wie in SEQ ID No. 1 dargestellt.

**5.** Replizierbare DNA gemäß Anspruch 2, enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

**6.** Replizierbare DNA gemäß Anspruch 5, **dadurch gekennzeichnet, daß** die Hybridisierung von Sequenz (iii) unter einer Stringenz entsprechend höchstens 2x SSC durchgeführt wird.

**7.** Polynukleotidsequenz gemäß Anspruch 2, die für ein Polypeptid kodiert, das die in SEQ ID No. 2 dargestellte Aminosäuresequenz enthält.

**8.** Coryneforme Bakterien, in denen das endogene sigD-Gen verstärkt, insbesondere überexprimiert wird.

**9.** Verfahren zur fermentativen Herstellung von L-Aminosäuren, insbesondere Lysin, **dadurch gekennzeichnet, daß** man folgende Schritte durchführt:
a) Fermentation der die gewünschte L-Aminosäure produzierenden coryneformen Bakterien, in denen man zumindest das endogene sigD-Gen oder dafür kodierende Nukleotidsequenzen verstärkt, insbesondere überexprimiert;
b) Anreicherung der L-Aminosäure im Medium oder in den Zellen der Bakterien, und
c) Isolierung der L-Aminosäure.

**10.** Verfahren gemäß Anspruch 9, **dadurch gekennzeichnet, daß** man Bakterien einsetzt, in denen man zusätzlich weitere Gene des Biosyntheseweges der gewünschten L-Aminosäure verstärkt.

**11.** Verfahren gemäß Anspruch 9, **dadurch gekennzeichnet, daß** man Bakterien einsetzt, in denen die Stoffwechselwege zumindest teilweise ausgeschaltet sind, die die Bildung der gewünschten L-Aminosäure verringern.

**12.** Verfahren gemäß Anspruch 9, **dadurch gekennzeichnet, daß** man einen mit einem Plasmidvektor transformierten Stamm einsetzt, und der Plasmidvektor die für das endogene sigD-Gen kodierende Nukleotidsequenz trägt.

**13.** Verfahren gemäß Anspruch 9, **dadurch gekennzeichnet, daß** man die Expression des (der) Polynukleotides (e), das (die) für das endogene sigD-Gen kodiert (kodieren) verstärkt, insbesondere überexprimiert.

**14.** Verfahren gemäß Anspruch 9, **dadurch gekennzeichnet, daß** man die regulatorischen Eigenschaften des Polypetids (Enzymprotein) erhöht, für das das endogene Polynukleotid sigD kodiert.

**15.** Verfahren gemäß Anspruch 9, **dadurch gekennzeichnet, daß** man zur Herstellung von L-Aminosäuren coryneforme Mikroorganismen fermentiert, in denen man gleichzeitig eines oder mehrere der Gene, ausgewählt aus der Gruppe
15.1 das für die Dihydrodipicolinat-Synthase kodierende Gen dapA,
15.2 das für die Glyceraldehyd-3-Phosphat Dehydrogenase kodierende Gen gap, fermentiert, in denen man gleichzeitig eines oder mehrere der Gene, ausgewählt aus der Gruppe
16.1 das für die Phosphoenolpyruvat-Carboxykinase kodierende Gen pck,
16.2 das für die Glucose-6-Phosphat Isomerase kodierende Gen pgi,
16.3 das für die Pyruvat-Oxidase kodierende Gen poxB
16.4 das für das Zwa2-Protein kodierende Gen zwa2 abschwächt.

**17.** Coryneforme Bakterien, die einen Vektor enthalten, der ein Polynukleotid gemäß Anspruch 1 trägt.

**18.** Verfahren gemäß einem oder mehreren der Ansprüche 9-16, **dadurch gekennzeichnet, daß** man Mikroorganismen der Gattung Corynebacterium einsetzt.

**19.** Verfahren zum Auffinden von RNA, cDNA und DNA, um Nukleinsäuren, beziehungsweise Polynukleotide oder Gene zu isolieren, die für den Sigma-Faktor D kodieren oder eine hohe Ähnlichkeit mit der Sequenz des sigD-Gens aufweisen, **dadurch gekennzeichnet, daß** man das Polynukleotid, enthaltend die Polynukleotidsequenzen gemäß den Ansprüchen 1, 2, 3 oder 4 als Hybridisierungssonden einsetzt.

**20.** Verfahren gemäß Anspruch 19, **dadurch gekennzeichnet, daß** man arrays, micro arrays oder DNA-chips einsetzt.


SEQUENZPROTOKOLL

<110> Degussa AG

<120> Für das sigD-Gen kodierende Nukleotidsequenzen

<130> 000443 BT

<140>
<141>

<160> 2

<170> PatentIn Ver. 2.1

<210> 1
<211> 1129
<212> DNA
<213> Corynebacterium glutamicum

<220>
<221> CDS
<222> (301)..(864)
<223> sigD-Gen

<400> 1
catatgcagg cgaactcctg ctagtacgcc cgttctgacc tgcggttatg tgtcgaggtg 60

aatctccggt gaattcttat agataacttg tttttgcagg tcaggacggg gttaagggga 120

tgggtgttat ctgtcagtat gtgaggagat caaggtgttg ggggttctag ttgctaagat 180

ggtgaaaacc cgtgaggcca aaatccaact gggtgaatta cccctgcata aatgcatgag 240

ggctttatac ttgtcttatt attaaacttt tagggttttg atgcaggaag gtgcgagaac 300

ttg gct gat act gag cgc gag ctc gct gac ctg gta ccg cag gca acg 348
Met Ala Asp Thr Glu Arg Glu Leu Ala Asp Leu Val Pro Gln Ala Thr
1               5                   10                  15

gcg ggc gat cgt cgg gca ttg caa aga ata atg gag att att cac ccc 396
Ala Gly Asp Arg Arg Ala Leu Gln Arg Ile Met Glu Ile Ile His Pro
            20                  25                  30

att gtt ttg cgt tat gct cgc gct cgt att gga ggt gga cgc cag cca 444
Ile Val Leu Arg Tyr Ala Arg Ala Arg Ile Gly Gly Gly Arg Gln Pro
        35                  40                  45

acg gca gaa gac gtt gct caa gaa atc tgc ctg gcg gta gcc acc tcc 492
Thr Ala Glu Asp Val Ala Gln Glu Ile Cys Leu Ala Val Ala Thr Ser
    50                  55                  60

att agg aac ttt gtc gac cag ggt agg ccg ttc atg gcg ttt gtc tac 540
Ile Arg Asn Phe Val Asp Gln Gly Arg Pro Phe Met Ala Phe Val Tyr
65                  70                  75                  80

ggc att gca tct aac aag gtc gca gat gct cac agg gcg atg tcg agg 588
Gly Ile Ala Ser Asn Lys Val Ala Asp Ala His Arg Ala Met Ser Arg
                85                  90                  95

gat aaa tcg act cct att gag gaa gtc cca gaa act tca cca gat act     636
Asp Lys Ser Thr Pro Ile Glu Glu Val Pro Glu Thr Ser Pro Asp Thr
            100                 105                 110

ttt acc ccc gaa gac ttt gcg ctg gtc agc gat gga agt aac aga gtt     684
Phe Thr Pro Glu Asp Phe Ala Leu Val Ser Asp Gly Ser Asn Arg Val
        115                 120                 125

agg gaa ctt ctc gat cta ctg agt gaa aag gca cgc gac att ctt atc     732
Arg Glu Leu Leu Asp Leu Leu Ser Glu Lys Ala Arg Asp Ile Leu Ile
    130                 135                 140

ttg aga gtt atc gtt ggt ctt tcc gca gaa gaa act gca gag atg gtg     780
Leu Arg Val Ile Val Gly Leu Ser Ala Glu Glu Thr Ala Glu Met Val
145                 150                 155                 160

ggc agc acc cca ggt gct gta cga gtt gcc caa cac agg gca ctc acg     828
Gly Ser Thr Pro Gly Ala Val Arg Val Ala Gln His Arg Ala Leu Thr
                165                 170                 175

aca ctt cga agc aca ctt gag cag cag gag aac aag taatgactcg          874
Thr Leu Arg Ser Thr Leu Glu Gln Gln Glu Asn Lys
            180                 185

acgtctacat ggtggtgagc aggatggcca ggaacacgtt aaaggacagc taaagcagct 934

gttcgacgac gacgcgttct tgactgacct gtcccgcggc gttgatccct cagagggcga 994

tgacgccctc gctggcctcc tcctcgattt aacaaaggaa gctcaggagc cgccggcaac 1054

aatgccggat tggtctactt tgctccctgg aattttggat caggatcagg atttgccagt 1114

ggaatccact tcgga                                                  1129


<210> 2
<211> 188
<212> PRT
<213> Corynebacterium glutamicum

<400> 2
Met Ala Asp Thr Glu Arg Glu Leu Ala Asp Leu Val Pro Gln Ala Thr
  1               5                  10                  15

Ala Gly Asp Arg Arg Ala Leu Gln Arg Ile Met Glu Ile Ile His Pro
             20                  25                  30

Ile Val Leu Arg Tyr Ala Arg Ala Arg Ile Gly Gly Gly Arg Gln Pro
         35                  40                  45

Thr Ala Glu Asp Val Ala Gln Glu Ile Cys Leu Ala Val Ala Thr Ser
     50                  55                  60

Ile Arg Asn Phe Val Asp Gln Gly Arg Pro Phe Met Ala Phe Val Tyr
 65                  70                  75                  80

Gly Ile Ala Ser Asn Lys Val Ala Asp Ala His Arg Ala Met Ser Arg
                 85                  90                  95

Asp Lys Ser Thr Pro Ile Glu Glu Val Pro Glu Thr Ser Pro Asp Thr
            100                 105                 110

Phe Thr Pro Glu Asp Phe Ala Leu Val Ser Asp Gly Ser Asn Arg Val
        115                 120                 125

Arg Glu Leu Leu Asp Leu Leu Ser Glu Lys Ala Arg Asp Ile Leu Ile
    130                 135                 140

Leu Arg Val Ile Val Gly Leu Ser Ala Glu Glu Thr Ala Glu Met Val
145                 150                 155                 160

Gly Ser Thr Pro Gly Ala Val Arg Val Ala Gln His Arg Ala Leu Thr
                165                 170                 175

Thr Leu Arg Ser Thr Leu Glu Gln Gln Glu Asn Lys
            180                 185